# EUROPEAN PATENT APPLICATION

(11) **EP 4 649 957 A1**
(43) Date of publication of application: **19.11.2025**
(21) Application number: 24175360.7
(22) Date of filing: 13.05.2024
(51) Int. Cl.: A61K 39/12, A61P 31/20

(54) **USE OF MRNA THAT ENCODES FLT3L FOR ENHANCING IMMUNE RESPONSES**

(71) Applicant: Universitätsmedizin der Johannes Gutenberg-Universität Mainz Körperschaft des öffentlichen Rechts, 55131 Mainz (DE)
(72) Inventor: GEHRING, Stephan, 55124 Mainz (DE); CACICEDO, Maximiliano Luis, 55118 Mainz (DE); LIMERES, María José, 80807 Munich (DE); SVENSSON, Malin, 55218 Ingelheim (DE)
(74) Representative: Krauss, Jan

(57) **Abstract**

The present invention relates to an mRNA encoding for an Flt3L protein or a functional fragment thereof for use in enhancing an immune response in a subject. The present invention further relates to a pharmaceutical composition, comprising the mRNA or the functional fragment thereof for use in medicine, in particular for a use in enhancing an immune response in a subject. The use comprises a combination prevention or therapy, such as, for example, a pre-treatment to improve vaccines against infectious diseases or cancer, or for enhancing an anticancer treatment in combination with at least one anticancer monoclonal antibody. The present invention further relates to a method for enhancing an immune response in a subject, comprising administering an effective amount of the mRNA encoding for an Flt3L protein or a functional fragment thereof according to the invention to the subject.

## Description

The present invention relates to an mRNA encoding for an Flt3L protein or a functional fragment thereof for use in enhancing an immune response in a subject. The present invention further relates to a pharmaceutical composition, comprising the mRNA or the functional fragment thereof for use in medicine, in particular for a use in enhancing an immune response in a subject. The use comprises a combination prevention or therapy, such as, for example, a pre-treatment to improve vaccines against infectious diseases or cancer, or for enhancing an anticancer treatment in combination with at least one anticancer monoclonal antibody. The present invention further relates to a method for enhancing an immune response in a subject, comprising administering an effective amount of the mRNA encoding for an Flt3L protein or a functional fragment thereof according to the invention to the subject.

### Background of the invention

Many diseases like cancer or chronic infectious diseases are difficult to treat with conventional vaccine-like approaches due to tolerogenic responses that impair the awakening of the immune system.

The hematopoietic cytokine fms-like tyrosine kinase 3 ligand (Flt3L) is a growth factor that has been widely identified as a strong stimulator in the generation of B cells and dendritic cells (DCs) (Dolence et al., 2014; Gilliet et al., 2002; Lin et al., 2018). Flt3L in protein form has proven to be a suitable adjuvant to enhance immune responses by recruiting DCs and B cells. *In vivo* administration in mice has shown a strong increase in both plasmacytoid and conventional DC populations (Fichter et al., 2016; Gehring et al., 2008; Tsapogas et al., 2017). Flt3L has also been studied in pre-clinical mouse models as pre-treatment to improve vaccine approaches against infectious diseases and cancer. Furthermore, Flt3L is being tested in clinical trials to enhance anticancer treatment together with monoclonal antibodies.

Flt3L administration has been suspected to be also responsible for the expansion in the numbers of natural killer (NK) cells and T lymphocytes (Solheim et al., 2007).

Kreiter et al. (in: FLT3 Ligand as a Molecular Adjuvant for Naked RNA Vaccines. Methods Mol Biol. 2016;1428:163-75. doi: 10.1007/978-1-4939-3625-0_11. PMID: 27236799) disclose that the intranodal immunization with antigen-encoding naked mRNA has proven to be an efficacious and safe approach to induce antitumor immunity. Thanks to its unique characteristics, mRNA can act not only as a source for antigen but also as an adjuvant for activation of the immune system. The search for additional adjuvants that can be combined with mRNA to further improve the potency of the immunization revealed Fms-like tyrosine kinase 3 (FLT3) ligand as a potent candidate. Systemic administration of the dendritic cell-activating FLT3 ligand prior to or along with mRNA immunization-enhanced priming and expansion of antigen-specific CD8(+) T cells in lymphoid organs, T-cell homing into melanoma tumors, and therapeutic activity of the intranodally administered mRNA. Both compounds demonstrate a successful combination in terms of boosting the immune response. This chapter describes methods for intranodal immunization with naked mRNA by co-administration of FLT3 ligand, which leads to strong synergistic effects.

Interestingly, Flt3L pre-treatment was also tested as a potentiator in a response against infectious diseases. In the search for successful treatments against challenging viral infections such as respiratory syncytial virus (RSV), lethal herpes simplex virus 1 (HSV-1), human immunodeficiency virus (HIV), or enterovirus A71, researchers concluded that Flt3L exerts protection mainly through robust activation of innate immune responses, type I IFNs, pDCs and B cells (Lin et al., 2018; Pham et al., 2019; Remot et al., 2016; Smith et al., 2001).

To date, Flt3L has always been used in form of protein-based formulations. In addition, recombinant human Flt3L when administered as a protein, has shown positive results in human trials regarding tolerability and effectivity when tested together with anti-cancer therapies. Clinical trials exposed positive results regarding the effective peripheral expansion of monocytes, hematopoietic stem cells and progenitor cells, and subsets of myeloid DCs and plasmacytoid DCs (Anandasabapathy et al., 2015; Bhardwaj et al., 2020).

EP3990476A1 discloses FLT3L-Fc fusion proteins, polynucleotides encoding such fusion proteins, expression cassettes, vectors, cells, and kits comprising such fusion proteins, and methods of use.

Despite the above success, formulations based on recombinant proteins are usually associated with side effects, such as anaphylactic responses due mainly to their production in cell cultures and to the lack of innate host post-translational modifications. Additionally, often antibodies against the therapeutic recombinant protein are found in serum after treatment. These issues, not only induce secondary adverse events but also reduce the therapeutic efficiency of the treatment. Furthermore, the production of recombinant proteins is time-consuming and expensive.

It is therefore an object of the present invention to provide new strategies to effectively provide Flt3L in the context of diseases and conditions, where an enhancement of the immune response is desired while avoiding the above problems and side effects. Other objects and advantages will become readily apparent to the person of skill when reading the present specification.

The problem of the present invention is solved by providing an RNA encoding for a Flt3L protein or a functional fragment thereof for use in enhancing an immune response in a subject.

Preferred is the RNA or the functional fragment thereof for use according to the present invention, wherein the RNA or the functional fragment thereof is naked RNA or wherein the RNA or the functional fragment thereof is part of a lipid nanoparticle (LNP), a polymeric nanoparticle, a polyplex, or a lipoplex, comprising said RNA or the functional fragment thereof.

Further preferred is the RNA or the functional fragment thereof for use according to the present invention, wherein the RNA or the functional fragment thereof is an mRNA molecule, a circular RNA molecule, or a self-amplifying RNA molecule.

The problem of the present invention is further solved by providing an expression cassette or vector, comprising an RNA and/or a DNA encoding for an RNA, preferably a mRNA, encoding for an Flt3L protein or a functional fragment thereof for use in enhancing an immune response in a subject.

The problem of the present invention is further solved by providing a pharmaceutical composition, comprising the RNA or the functional fragment thereof or the LNP for use according to the present invention, the expression cassette or vector for use according to the present invention and at least one pharmaceutically acceptable carrier for use in medicine, in particular for a use in enhancing an immune response in a subject, wherein preferably said composition comprises an aqueous formulation.

Preferably, enhancing an immune response according to the present invention in a subject comprises the use of the pharmaceutical composition according to the present invention as an adjuvant, e.g. in a vaccine, in particular as an anti-cancer or anti-infective adjuvant, as a suitable adjuvant enhancing immune responses by recruiting DCs and B cells, for an expansion of the numbers of natural killer (NK) cells, DCs and/or T lymphocytes, for priming and expansion of antigen-specific CD8⁺ T cells, an activation of T cells, and/or an increase in cytokine-secreting CD4+ T cells and/or for improving the T-cell homing into melanoma tumors. Further preferred uses of the pharmaceutical composition according to the present invention comprise use in a combination prevention or therapy, such as, for example, a pre-treatment to improve vaccines against infectious diseases or cancer, or for enhancing an anticancer treatment in combination with at least one anticancer monoclonal antibody.

Further preferred is the pharmaceutical composition for use according to the present invention, wherein the subject suffers from cancer or infection, in particular bacterial or viral infection.

Further preferred is the pharmaceutical composition for use according to the present invention, wherein the use comprises administration intravenously (i.v.), intranodally (i.n.), and/or intramuscular (i.m.), preferably together with another RNA encoding for a protein antigen or adjuvants.

The problem of the present invention is further solved by providing a method for enhancing an immune response in a subject, comprising administering an effective amount of an RNA, preferably an mRNA, encoding for an Flt3L protein or a functional fragment thereof to said subject. Preferred is the method according to the present invention, wherein the RNA or the functional fragment thereof is naked RNA, or wherein the RNA or the functional fragment thereof is part of a lipid nanoparticle (LNP), a polymeric nanoparticle, a polyplex, or a lipoplex, comprising said RNA or the functional fragment thereof.

Further preferred is the method for enhancing an immune response in a subject according to the present invention, wherein the RNA or the functional fragment thereof is an mRNA molecule, a circular RNA molecule, or a self-amplifying RNA molecule.

The problem of the present invention is further solved by a method for enhancing an immune response in a subject according to the present invention, comprising administering an effective amount of an expression cassette or vector comprising the RNA and/or a DNA encoding for the RNA, preferably an mRNA, encoding for an Flt3L protein or a functional fragment thereof to said subject, wherein preferably the vector is a plasmid vector or a viral vector.

The problem of the present invention is further solved by a method for enhancing an immune response in a subject according to the present invention, wherein the RNA or the functional fragment thereof, the LNP, the expression cassette or the vector is administered as a pharmaceutical composition, further comprising at least one pharmaceutically acceptable carrier, preferably as an anti-infective or anti-cancer vaccine.

Preferred is the method according to the present invention, wherein said method comprises the use as an adjuvant, in particular as an anti-cancer or anti-infective adjuvant, as a suitable adjuvant enhancing immune responses by recruiting DCs and B cells, for an expansion of the numbers of natural killer (NK) cells, DCs and/or T lymphocytes, for priming and expansion of antigen-specific CD8⁺ T cells, an activation of T cells, and/or an increase in cytokine-secreting CD4+ T cells for improving an anti-infective or anti-cancer effect of an intranodal RNA or an RNA-based intranodal vaccine, for improving the T-cell homing into tumors, and/or the use comprises a combination prevention or therapy, such as, for example, a pre-treatment to improve vaccines against infectious diseases or cancer, or for enhancing an anticancer treatment in combination with at least one anti-infective or anticancer monoclonal antibody. The expansion of DCs by Flt3L-mRNA has been observed in liver, spleen and iLNs.

Further preferred is the method according to the present invention, wherein the subject suffers from cancer or infection, in particular bacterial or viral infection.

Further preferred is the method according to the present invention, comprising administration i.v., i.n., and/or i.m., preferably together with another RNA encoding for a protein antigen or adjuvants.

As described above, in a first aspect thereof, the present invention solves the above object by providing an RNA encoding for an Flt3L protein or a functional fragment thereof for use in enhancing an immune response in a subject.

In the context of the present invention, the term "Flt3L protein" shall generally relate to the mammalian protein of Flt3L, in particular human Flt3L protein. The amino acid sequence of the human protein can be found at number P49771 in the UniProt database. Particularly included are the two isoforms of Flt3L, i.e., the membrane-bound isoform 1, and the soluble isoform 2, lacking the C-terminal sequence after amino acid 178 (in humans). Both proteins may form homodimers. The term further includes Flt3L proteins that are identical or at least 80%, preferably at least 90%, more preferably at least 95% identical to the amino acid sequence of the mammalian Flt3L protein, in particular the human protein.

In the context of the present invention, the term "functional fragment of the Flt3L protein" shall relate to a part of the amino acid sequence of the above Flt3L protein that is truncated at the N- and/or C-terminus of the amino acid sequence, but still performs its function as required/desired in the context of the present invention, namely - when expressed, translated and/or administered - enhances an immune response in a subject. The N-terminal truncation may be amino acids 1 to 27, and the C-terminal truncation may be amino acids 179 to 235 (in humans). As described herein, examples of more specific functions are the effect of an adjuvant, in particular as an anti-cancer or anti-infective adjuvant, as a suitable adjuvant enhancing immune responses by recruiting DCs and B cells, for an expansion of the numbers of natural killer (NK) cells, DCs and/or T lymphocytes, for priming and expansion of antigen-specific CD8⁺ T cells, an activation of T cells, and/or an increase in cytokine-secreting CD4+ T cells, and/or for improving the T-cell homing into tumors. Further functions are the effect that a pre-treatment improve vaccines against infectious diseases or cancer, and/or enhances an anticancer treatment in combination with at least one anticancer monoclonal antibody.

In the context of the present invention, the term "RNA" shall relate to a respective nucleic acid molecule comprising a nucleic acid sequence encoding for the Flt3L protein or functional fragment thereof as above. The RNA molecule can be natural or produced synthetically or in vitro, or combinations thereof. The RNA may comprise modified bases and/or DNA or other types of nucleic acid components, as long as the desired function(s) (see above) is/are not substantially impaired. In the context of the invention, so-called "naked RNA", circular RNA molecule (see, for example, Liu X, et al. Circular RNA: An emerging frontier in RNA therapeutic targets, RNA therapeutics, and mRNA vaccines. J Control Release. 2022 Aug;348:84-94. Doi: 10.1016/j.jconrel.2022.05.043. Epub 2022 Jun 2), or a self-amplifying RNA (see, for example, Lundstrom K. Self-Amplifying RNA Viruses as RNA Vaccines. Int J Mol Sci. 2020 Jul 20;21(14):5130. Doi: 10.3390/ijms21145130) molecule may be used, preferably an mRNA is used that comprises the same structural components as natural mRNA in eukaryotic cells. It has at least a 5' cap, a 5'-untranslated region (UTR), a 3'-UTR, an open reading frame_(ORF), which encodes the relevant Flt3L protein or functional fragment thereof, for example as adjuvant or antigen, and a 3'-poly(A) tail. Preferably, the term further includes RNAs comprising nucleotide sequences that are identical or at least 80%, preferably at least 90%, more preferably at least 95% identical to the nucleotide sequence of an RNA, such as an mRNA, encoding the mammalian Flt3L protein, in particular the human protein.

In an RNA vaccine, which is the preferred way of administration, the central component is the RNA or RNA construct. If the RNA is *in vitro* transcribed the RNA is generated from an engineered plasmid (vector) DNA, which has an RNA polymerase promoter_and the Flt3L protein sequence which is transcribed into the mRNA construct. By combining, e.g., T7 phage RNA polymerase and the plasmid DNA, the mRNA can be transcribed. The efficacy of the vaccine is further dependent on the stability and structure of the designed mRNA.

The person of skill is aware that by modifying the different components of the synthetic RNA, the stability and translational ability of the RNA can be enhanced, and in turn, the efficacy of a respective vaccine improved. For mRNA, such improvement can be achieved, for example, by using synthetic 5'-cap analogues that enhance the stability and increase protein translation. Similarly, regulatory elements in the 5'-UTR and the 3'-UTR can be altered, and the length of the poly(A) tail optimized, to stabilize the mRNA and increase protein production. The RNA nucleotides can be modified to both decrease innate immune activation and increase the mRNA's half-life in the host cell (see also above). The nucleic acid sequence and codon usage impact protein translation. Enriching the sequence with guanine-cytosine content will usually improve RNA stability and half-life and, in turn, protein production. Replacing rare codons with synonymous codons frequently used by the host cell also enhances protein production. All these modifications are well within the skill of the person of skill in the art.

In the context of the present invention, a "subject" relates to an animal or individual, such as a person or patient that undergoes prophylaxis or treatment according to the invention in order to enhance immune responses.

Preferred is then the RNA or the functional fragment thereof for use according to the present invention, wherein the RNA or the functional fragment thereof is naked RNA. The naked RNA is usually injected, and this means that the delivery of the vaccine is only done in a suitable buffer solution. A variety of methods have been used to deliver naked RNA or naked mRNA, such as subcutaneous, intravenous, and intratumoral injections.

Further preferred is the RNA or the functional fragment thereof for use according to the present invention, wherein the RNA or the functional fragment thereof is suitably coated, complexed and/or part of a lipid nanoparticle (LNP), a polymeric nanoparticle, a polyplex, or lipoplex comprising said RNA or the functional fragment thereof. Cationic polymers can be mixed with RNA to generate protective coatings called polyplexes. These protect the recombinant RNA from ribonucleases and assist its penetration in cells. Protamine is a natural cationic peptide and can be used to encapsulate RNA for vaccination. Further provided is a lipoplex, such as a lipid nanoparticle (LNP), comprising an RNA encoding the FLT3L protein described herein for use according to the present invention.

Eygeris Y, et al. (in: Chemistry of Lipid Nanoparticles for RNA Delivery. Acc Chem Res. 2022 Jan 4;55(1):2-12. Doi: 10.1021/acs.accounts.lc00544. Epub 2021 Dec 1. PMID: 34850635) describe the use of lipid nanoparticles (LNPs) in nucleic acid delivery and vaccines against infectious diseases, and cancer vaccines. Encapsulation of mRNA within LNPs protects mRNA from extracellular ribonucleases and assists with intracellular mRNA delivery. They highlight the most recent clinical successes of LNP-based nucleic acid therapies and describe the theory and methods of LNP self-assembly. Then, they break down the LNP composition with special attention to the fundamental properties and purposes of each component and review the attributes of LNP constructs as a whole that can heavily influence RNA delivery.

Another aspect of the present invention the relates to an expression cassette or vector, comprising an mRNA and/or a DNA encoding for an mRNA encoding for an Flt3L protein or a functional fragment thereof for use in enhancing an immune response in a subject. The expression cassette comprises one or more prokaryotic or eukaryotic regulatory sequences operably linked to the mRNA or DNA. Preferred is the expression cassette or vector for use according to the present invention, wherein the vector is a plasmid vector or a viral vector. The viral vector may comprise the expression cassette, the viral vector may comprise an oncolytic viral vector or the viral vector comprises a DNA virus or a RNA virus. The viral vector can be selected from a viral family selected from the group consisting of Adenoviridae (e.g., Adenovirus), Poxviridae (e.g., Vaccinia virus), Herpesviridae (e.g., Herpesvirus, e.g., HSV-1), Parvoviridae (e.g., Parvovirus H1), Reoviridae (e.g., Reovirus), Togaviridae (e.g., Alphavirus, Sindbis virus), and Enteroviridae (e.g., Echovirus).

It was surprisingly found that the administration of the mRNA and respective formulations were not associated with side effects, such as anaphylactic responses. Additionally, no substantial antibodies against the therapeutic recombinant protein could be found, thus ensuring the full therapeutic efficiency of the treatment.

Administration of the present mRNA-based approach will in one embodiment guarantee a more tolerable and patient-friendly strategy to boost immunity before formal vaccination against infectious diseases or before immunotherapy.

Another aspect of the present invention the relates to a pharmaceutical composition comprising the mRNA or the functional fragment thereof or the LNP for use according to the present invention, the expression cassette or vector for use according to the present invention and at least one pharmaceutically acceptable carrier for use in medicine. Preferred is a pharmaceutical composition, comprising the mRNA or the functional fragment thereof or the LNP for use according to the present invention, the expression cassette or vector for use according to the present invention and at least one pharmaceutically acceptable carrier for use in enhancing an immune response in a subject.

Preferably, said composition comprises an aqueous formulation. The term "pharmaceutical composition" refers to a preparation which is in such form as to permit the biological activity of an active ingredient contained therein (here, the mRNA) to be effective, and which contains no additional components which are unacceptably toxic to a subject to which the composition would be administered. A pharmaceutical composition of the present invention can be administered by a variety of methods known in the art. As will be appreciated by the skilled artisan, the route and/or mode of administration will vary depending upon the desired results.

Pharmaceutically acceptable diluents include saline and aqueous buffer solutions. A "pharmaceutically acceptable carrier" refers to an ingredient in a pharmaceutical formulation, other than an active ingredient, which is nontoxic to a subject. Pharmaceutically acceptable carriers include any and all solvents, dispersion media, coatings, antibacterial and antifungal agents, isotonic and absorption delaying agents, and the like that are physiologically compatible. The carrier can be suitable for intravenous, intramuscular, subcutaneous, parenteral, spinal or epidermal administration (e.g. by injection or infusion).

The pharmaceutical compositions according to the invention may also contain adjuvants such as preservatives, wetting agents, emulsifying agents and dispersing agents. Prevention of presence of microorganisms may be ensured both by sterilization procedures and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

The pharmaceutical compositions according to the invention may be in liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, plant juice, nasal agent, inhalation mixture, aerosol, mouthwash, mouth spray, nose spray, or room spray. Preferred is an injectable composition.

In some embodiments, the composition comprises the active ingredient, such as the mRNA-containing LNP at a concentration in the range of about 1 mg/ml to about 2 mg/ml, 3 mg/ml, 4 mg/ml, 5 mg/ml, 6 mg/ml, 7 mg/ml, 8 mg/ml, 9 mg/ml, 10mg/ml, 11 mg/ml, 12 mg/ml, 13 mg/ml, 14 mg/ml, 15 mg/ml, 16 mg/ml, 17 mg/ml, 18 mg/ml, 19 mg/ml or 20 mg/ml. In some embodiment, the composition further comprises one or more additional therapeutic agents, e.g., second, third or fourth therapeutic agents.

According to this aspect of the pharmaceutical composition for use according to the present invention, enhancing an immune response in a subject preferably comprises a use as an adjuvant, in particular as an anti-cancer or anti-infective adjuvant in order to increase the efficacy or potency of a primary therapy and/or to increase the response of the immune response as produced by the subject. Preferred sub-aspects and uses include use as a suitable adjuvant enhancing immune responses by recruiting DCs and B cells, for an expansion of the numbers of natural killer (NK) cells, DCs and/or T lymphocytes, for priming and expansion of antigen-specific CD8⁺ T cells, an activation of T cells, and/or an increase in cytokine-secreting CD4+ T cells, and/or for improving the T-cell homing into tumors.

The pharmaceutical composition for use according to the present invention is preferably employed in a combination therapy or prevention. Preferred examples are pre-treatment to "prime" the immune response, or to improve the effect of vaccines against infectious diseases or cancer. Another aspect is enhancing an anticancer treatment, for example in combination with at least one anticancer monoclonal antibody. Preferred is the pharmaceutical composition for use according to the present invention, wherein the subject suffers from cancer or infection, in particular bacterial or viral infection. Flt3L pre-treatment was tested as a potentiator in a response against infectious diseases. In the search for successful treatments against challenging viral infections such as respiratory syncytial virus (RSV), lethal herpes simplex virus 1 (HSV-1), human immunodeficiency virus (HIV), or enterovirus A71, researchers concluded that Flt3L exerts protection mainly through robust activation of innate immune responses, type I IFNs, pDCs and B cells ((Lin et al., 2018; Pham et al., 2019; Remot et al., 2016; Smith et al., 2001).

Further preferred is the pharmaceutical composition for use according to the present invention, wherein the use comprises administration i.v., i.n., and/or i.m., preferably together with another RNA encoding for a protein antigen or adjuvants.

The combination can be administered together or in separate doses, as long as the effects of the pharmaceuticals lead to a combinatory effect of the drugs as applied.

Yet another important aspect of the present invention relates to a method for enhancing an immune response in a subject, comprising administering an effective amount of an mRNA encoding for an Flt3L protein or a functional fragment thereof to said subject.

Preferred is method according to the present invention, wherein the mRNA or the functional fragment thereof is naked mRNA. The naked mRNA is usually injected, and this means that the delivery of the vaccine is only done in a suitable buffer solution. A variety of methods have been used to deliver naked mRNA, such as subcutaneous, intravenous, intranodal, intramuscular and/or intratumoral injections.

Further preferred is the method according to the present invention, wherein the RNA or the functional fragment thereof is suitably coated, complexed and/or part of a lipid nanoparticle (LNP), a polymeric nanoparticle, a polyplex, or lipoplex comprising said RNA or the functional fragment thereof.

Another aspect of the present invention the relates to the method according to the present invention, comprising administering to said subject an effective amount of an expression cassette or vector comprising the mRNA and/or a DNA encoding for the mRNA encoding for an Flt3L protein or a functional fragment thereof, wherein preferably the vector is a plasmid vector or a viral vector.

Administration of the present RNA-based approach will in one embodiment guarantee a more tolerable and patient-friendly strategy to boost immunity before formal vaccination against infectious diseases or before immunotherapy.

Another aspect of the present invention the relates to the method according to the present invention, wherein the RNA or the functional fragment thereof, the LNP, the polymeric nanoparticle, the polyplex, the lipoplex, the expression cassette or the vector is administered as a pharmaceutical composition, further comprising at least one pharmaceutically acceptable carrier as described above, preferably as an anti-cancer vaccine.

According to this aspect of the method according to the present invention, enhancing an immune response in a subject preferably comprises a use as an adjuvant, in particular as an anti-cancer or anti-infective adjuvant in order to increase the efficacy or potency of a primary therapy and/or to increase the response of the immune response as produced by the subject. Preferred sub-aspects and uses include the use as a suitable adjuvant enhancing immune responses by recruiting DCs and B cells, the expansion of the numbers of natural killer (NK) cells, DCs and/or T lymphocytes, for priming and expansion of antigen-specific CD8⁺ T cells, an activation of T cells, and/or an increase in cytokine-secreting CD4+ T cells, and/or for improving the T-cell homing into tumors.

Preferred is the method according to the present invention, wherein the subject suffers from cancer or infection, in particular bacterial or viral infection.

Preferred is the method according to the present invention, comprising administration i.v., i.n., and/or i.m., preferably together with another RNA encoding for a protein antigen or adjuvants.

Further provided are methods of promoting, inducing and/or increasing the expansion and/or proliferation of a cell or a population of cells that express fms related tyrosine kinase 3 (FLT3, CD135), comprising contacting the cell or population of cells with an effective amount of a pharmaceutical composition as described herein; a polynucleotide encoding a FLT3L protein as described herein, or an expression cassette, vector, lipoplex, such as an LNP. In some embodiments, the cell or population of cells that express FLT3 comprise dendritic cells (e.g., cDC1 cells and/or cDC2 cells), monocyte-derived dendritic cells (moDCs), and/or progenitor cells thereof. In some embodiments, the cell or population of cells that express FLT3 comprise hematopoietic progenitor cells.

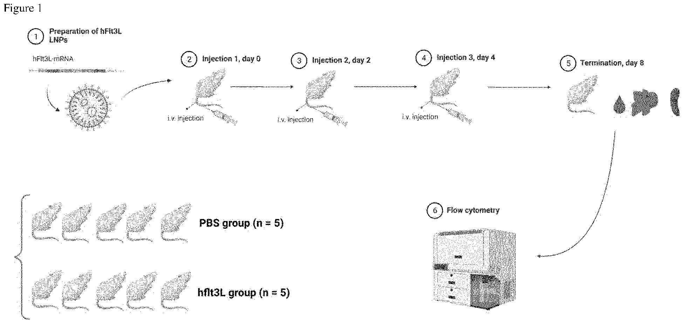

As the main professional antigen-presenting cells, DCs are critical for the initiation of the primary immune responses by eliciting T-cell immunity. Thus, targeting the DC population represents a highly desirable characteristic in vaccination strategies. Furthermore, expanding DC subsets by administration of Flt3L has the potential to generate a considerable improvement in vaccine-induced immune responses. The inventors have developed a treatment based on Flt3L-encoding mRNA formulated in lipid nanoparticles. Once administered through intravenous injection, the mRNA is translated into Flt3L protein and exerts the typical immunostimulatory properties described in the literature. Figure 1 summarizes the experimental design performed to test this mRNA-based formulation *in vivo.*

mRNA-based approaches allow the intracellular expression of the therapeutic protein using the cellular machinery of the host, leading to the preservation of the innate post-translational modifications. This way, challenges often encountered with protein-based drugs, such as anaphylactic reactions and delivery issues, can be addressed. Additionally, mRNA-based formulations include cell-free manufacturing processes and no risk of insertional mutagenesis like DNA-based therapies (Kowalski et al., 2019).

Administration of the present RNA-based approach will guarantee a more tolerable and patient-friendly strategy to boost immunity before formal vaccination against infectious diseases or before immunotherapy.

The mRNA technology has proven to have a huge potential to replace any protein-related treatment. mRNA production is simple, comparatively cheap, and can be achieved very fast. The therapeutic protein is obtained after the mRNA is translated using the cellular machinery from the host. Thus, this generates a protein with a native structure avoiding anaphylactic responses. Moreover, since mRNA constructs are optimized, large amounts of therapeutic protein can be obtained improving the therapeutic outcome.

The present invention relates to the following items:
Item 1. An RNA encoding for an Flt3L protein or a functional fragment thereof for use in enhancing an immune response in a subject.
Item 2. The mRNA or the functional fragment thereof for use according to Item 1, wherein the RNA or the functional fragment thereof is naked RNA, an mRNA molecule, a circular RNA molecule, or a self-amplifying RNA molecule.
Item 3. The mRNA or the functional fragment thereof for use according to Item 1 or 2, wherein the RNA or the functional fragment thereof is part of a lipid nanoparticle (LNP), a polymeric nanoparticle, a polyplex, or a lipoplex, comprising said RNA or the functional fragment thereof.
Item 4. An expression cassette or vector, comprising an RNA and/or a DNA encoding for an RNA, preferably an mRNA, encoding for an Flt3L protein or a functional fragment thereof for use in enhancing an immune response in a subject.
Item 5. The expression cassette or vector for use according to Item 4, wherein the vector is a plasmid vector or a viral vector.
Item 6. A pharmaceutical composition, comprising the RNA or the functional fragment thereof or the LNP for use according to any one of Items 1 to 3, the expression cassette or vector for use according to Item 4 or 5 and at least one pharmaceutically acceptable carrier for use in medicine, in particular for a use in enhancing an immune response in a subject, wherein preferably said composition comprises an aqueous formulation.
Item 7. The pharmaceutical composition for use according to Item 6, wherein said enhancing an immune response in a subject comprises a use as an adjuvant, in particular as an anti-cancer or anti-infective adjuvant, as a suitable adjuvant enhancing immune responses by recruiting DCs and B cells, for an expansion of the numbers of natural killer (NK) cells, DCs and/or T lymphocytes, for priming and expansion of antigen-specific CD8⁺ T cells, an activation of T cells, and/or an increase in cytokine-secreting CD4+ T cells.
Item 8. The pharmaceutical composition for use according to Item 6, wherein said use comprises a combination prevention or therapy, such as, for example, a pre-treatment to improve vaccines against infectious diseases or cancer, or for enhancing an anti-infective or anticancer treatment in combination with at least one anti-infective or anticancer monoclonal antibody.
Item 9. The pharmaceutical composition for use according to any one of Items 6 to 8, wherein the subject suffers from cancer or infection, in particular bacterial or viral infection.
10. The pharmaceutical composition for use according to any one of Items 6 to 8, wherein the use comprises administration i.v., i.n., and/or i.m., preferably together with another RNA encoding for a protein antigen or adjuvants.
Item 11. A method for enhancing an immune response in a subject, comprising administering an effective amount of an RNA encoding for an Flt3L protein or a functional fragment thereof to said subject.
Item 12. The method according to Item 11, wherein the RNA or the functional fragment thereof is naked mRNA, an mRNA molecule, a circular RNA molecule, or a self-amplifying RNA molecule.
Item 13. The method according to Item 11, wherein the RNA or the functional fragment thereof is part of a lipid nanoparticle (LNP), a polymeric nanoparticle, a polyplex, or a lipoplex, comprising said RNA or the functional fragment thereof.
Item 14. The method according to Item 11, comprising administering an effective amount of an expression cassette or vector comprising the RNA and/or a DNA encoding for the RNA, preferably the mRNA, encoding for an Flt3L protein or a functional fragment thereof to said subject, wherein preferably the vector is a plasmid vector or a viral vector.
Item 15. The method according to any one of Items 11 to 14, wherein the RNA or the functional fragment thereof, the LNP, the expression cassette or the vector is administered as a pharmaceutical composition, further comprising at least one pharmaceutically acceptable carrier, preferably as an anti-infective or anti-cancer vaccine.
Item 16. The method according to Item 15, wherein said method comprises the use as an adjuvant, in particular as an anti-cancer or anti-infective adjuvant, as a suitable adjuvant enhancing immune responses by recruiting DCs and B cells, for an expansion of the numbers of natural killer (NK) cells, DCs and/or T lymphocytes, for priming and expansion of antigen-specific CD8⁺ T cells, an activation of T cells, and/or an increase in cytokine-secreting CD4+ T cells, and/or the use comprises a combination prevention or therapy, such as, for example, a pre-treatment to improve vaccines against infectious diseases or cancer, or for enhancing an anticancer treatment in combination with at least one anticancer monoclonal antibody.
Item 17. The method according to any one of Items 11 to 16, wherein the subject suffers from cancer or infection, in particular bacterial or viral infection.
Item 18. The method according to any one of Items 11 to 17, wherein the method comprises an administration i.v., i.n., and/or i.m., preferably together with another RNA encoding for a protein antigen or adjuvants.

The invention will now be described further in the following examples with reference to the accompanying figures, nevertheless, without being limited thereto. For the purposes of the present invention, all references as cited are incorporated by reference in their entireties.
Figure 1 shows the effects of *hFlt3L-mRNA* LNPs at a cellular level after *in vivo* administration.
Figure 2 shows the analysis of the different DCs subpopulations within (A) the spleen, (B) the liver non-parenchymal cells (NPCs), (C) and inguinal Lymph nodes (iLNs).
Figure 3 shows the analysis of *hFlt3L-mRNA* co-encapsulated with an antigen-encoding mRNA (HBsAg) that activates CD4+ and CD8+ T cells.
Figure 4 shows analysis of *hFlt3L-mRNA* co-encapsulated with an antigen-encoding mRNA (HBsAg) improves specific immune responses after i.m. administration.

### Examples

*hFlt3L-mRNA* was formulated using commercial LNPs and was administered i.v. every two days with a total of 3 doses. On day 8, animals were euthanized, and blood, spleen, liver and iLNs were harvested to perform flow cytometry analyzes.

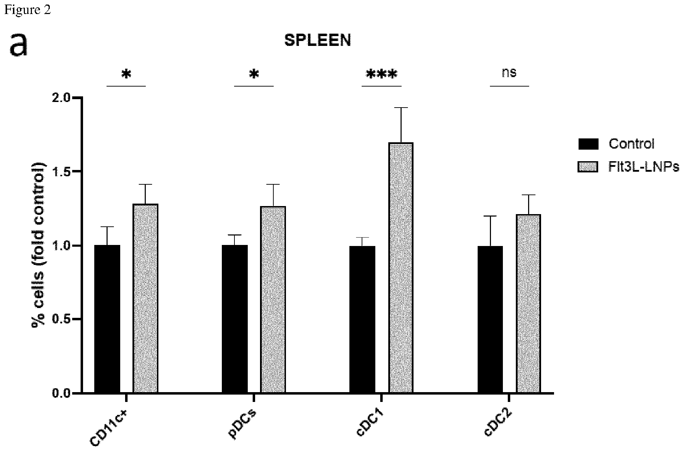

Results showed that in the spleen of the *hFlt3L*-mRNA-treated animals, the CD11c⁺ population was significantly increased (Figure 2A). CD11c is used as a marker to identify the total DCs population. When studying in detail each DC subtype, plasmacytoid DCs (pDCs) and conventional DCs type I (cDC1) were shown to be remarkably increased. However, conventional DCs type II (cDC2) did not show differences with untreated animals.

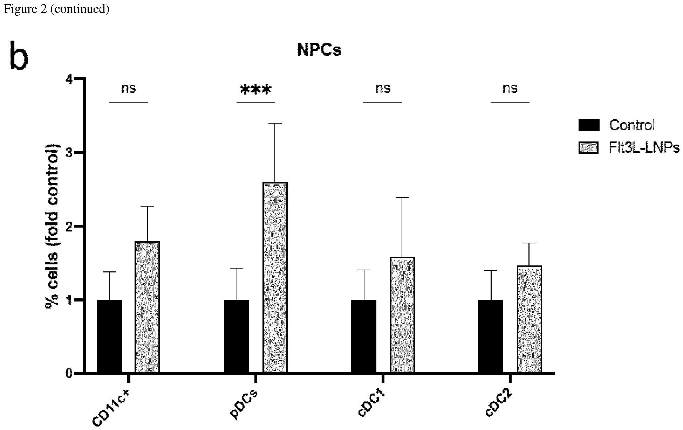

In the case of the liver, only a robust increase in pDCs was found (Figure 2B). Although no significant differences were found for the other cell types, a marked increase in tendency was observed after the treatment.

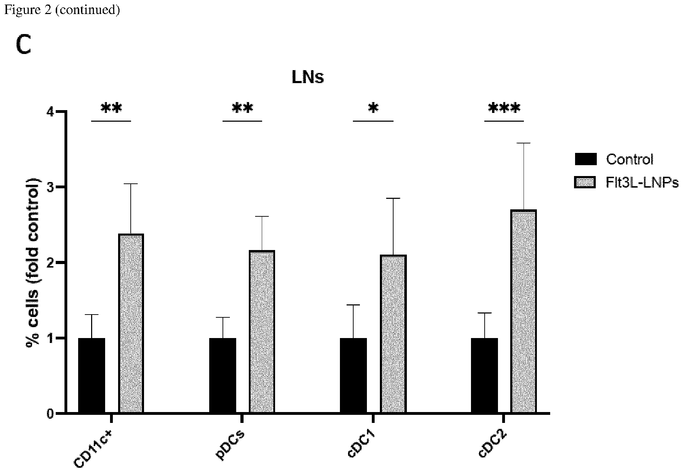

Notably, strong increments in each DC subtype were observed in the case of inguinal lymph nodes (iLNs) (Figure 2C). This is of particular interest since LNs work as centers for immune cell recruitment. In summary, the inventor's *hFlt3L-mRNA* formulation proved to be highly effective in the increment of DC populations within immunologically relevant organs.

The dosage and frequency of the administration can be adjusted if a stronger effect is needed.

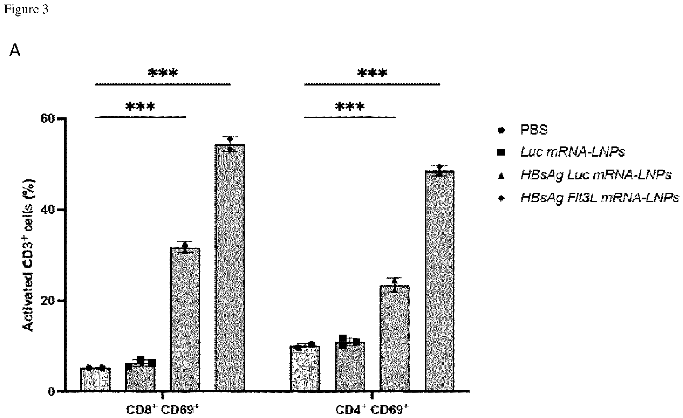

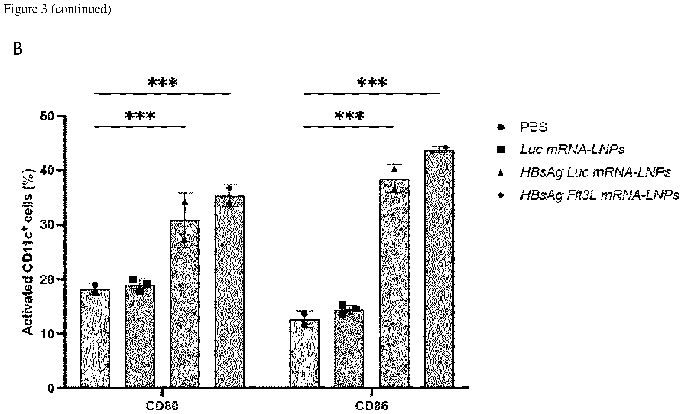

Another remarkable use of *hFlt3L-mRNA* was found after its administration in combination with a model mRNA-encoded antigen, such as Hepatitis B surface antigen (HBsAg). In the experiment shown in Figure 3, *hFlt3L-mRNA* was co-encapsulated with HBsAg-mRNA and administered by intramuscular (i.m.) injection. To evaluate the adjuvant effects that *hFlt3L-*mRNA could provoke, early activation markers were analyzed in spleen cells 24 h after injection. Activation of both DCs and T cells represents a fundamental objective for a vaccine to mount robust immune responses against the antigen. In this particular case, LNPs containing HBsAg-mRNA induce activation of T cells and DCs independently of the presence of *hFlt3L-*mRNA in the formulation (Figures 3A and B). However, when *hFlt3L-mRNA* was co-administered with HBsAg-mRNA it was observed a notable up-regulation in CD69 surface marker either for CD8+ or CD4+ T cells (Figure 3A). This finding potentiates the potential application of *hFlt3L-mRNA* as a genetic adjuvant for mRNA-based vaccines.

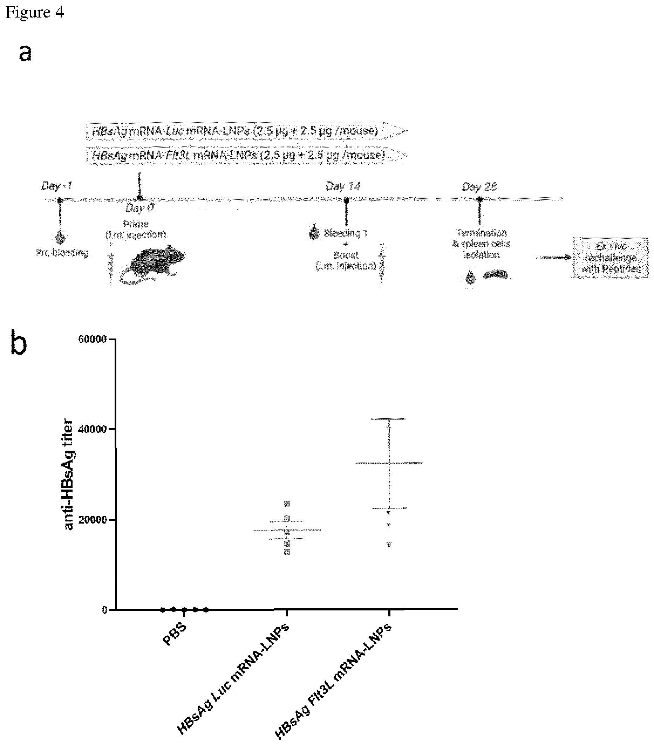

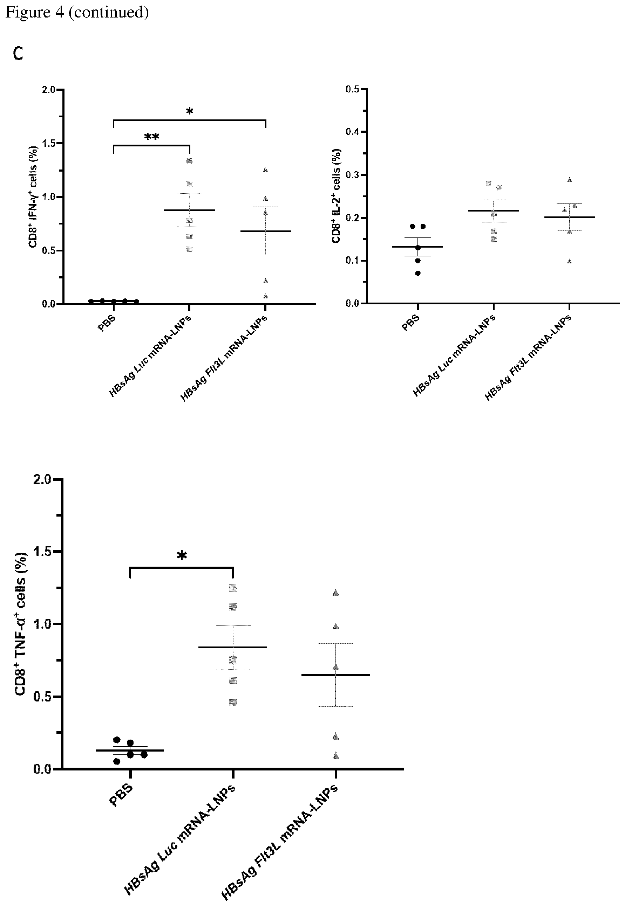

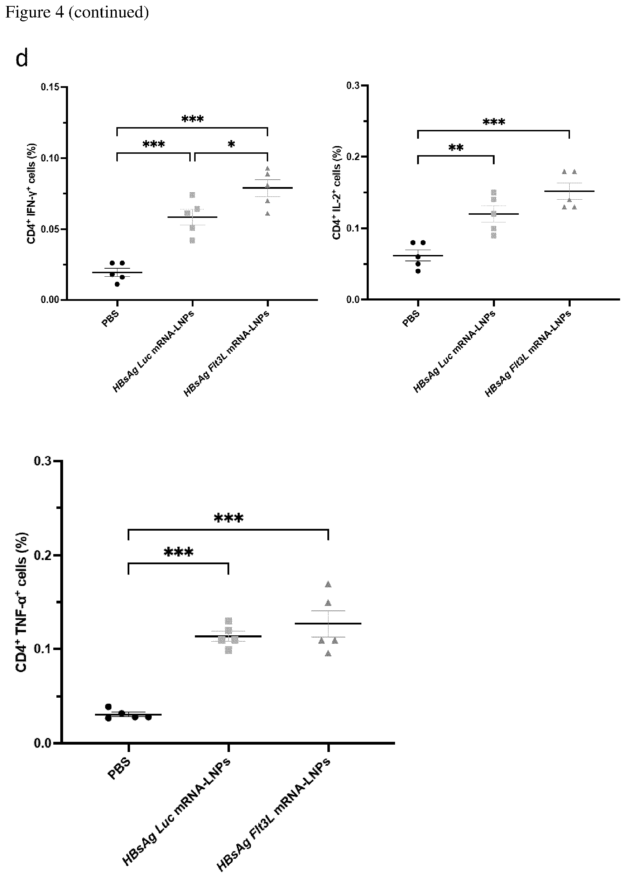

To continue with the inventor's understanding of the role of *hFlt3L-mRNA* as a genetic adjuvant, a combination with HBsAg-mRNA was applied in an immunization scheme in WT mice (Figure 4A). Experiment termination was set on day 28 after the first immunization. Results showed an increase in anti-HBsAg IgG titers when HBsAg-mRNA is applied together with *hFlt3L*-mRNA, in comparison to the control group (Figure 4B). Additionally, the *hFlt3L-*mRNA adjuvanted vaccine leads to a significant increase in cytokine-secreting CD4+ T cells (IFNa+, IL2+ and TNFa+) `(Figure 4D).

### References

Anandasabapathy, N., et al. (2015). Efficacy and safety of CDX-301, recombinant human Flt3L, at expanding dendritic cells and hematopoietic stem cells in healthy human volunteers. Bone Marrow Transplantation, 50(7), 924-930. https://doi.org/10.1038/bmt.2015.74
Bhardwaj, N., et al. (2020). Flt3 ligand augments immune responses to anti-DEC-205-NY-ESO-1 vaccine through expansion of dendritic cell subsets. Nature Cancer, 1(12), 1204-1217. https://doi.org/10.1038/s43018-020-00143-y
Dolence, J. J., Gwin, K. A., Shapiro, M. B., & Medina, K. L. (2014). Flt3 signaling regulates the proliferation, survival, and maintenance of multipotent hematopoietic progenitors that generate B cell precursors. Experimental Hematology, 42(5), 380-393.e3. https://doi.org/10.1016/j.exphem.2014.01.001
Fichter, M., et al. (2016). Polymeric hepatitis C virus non-structural protein 5A nanocapsules induce intrahepatic antigen-specific immune responses. Biomaterials, 108, 1-12. https://doi.org/10.1016/j.biomaterials.2016.08.046
Gehring, S., et al. (2008). Generation and characterization of an immunogenic dendritic cell population. Journal of Immunological Methods, 332(1-2), 18-30. https://doi.org/10.1016/j.jim.2007.12.007
Gilliet, M., et al. (2002). The Rockefeller University Press • 0022-1007. In J. Exp. Med (Vol. 195, Issue 7). http://www.jem.org/cgi/content/full/195/7/953
Kowalski, P. S., Rudra, A., Miao, L., & Anderson, D. G. (2019). Delivering the Messenger: Advances in Technologies for Therapeutic mRNA Delivery. In Molecular Therapy (Vol. 27, Issue 4, pp. 710-728). Cell Press. https://doi.org/10.1016/j.ymthe.2019.02.012
Kreiter, S., et al. (2011). FLT3 ligand enhances the cancer therapeutic potency of naked RNA vaccines. Cancer Research, 71(19), 6132-6142. https://doi.org/10.1158/0008-5472.CAN-11-0291
Lin, Y. W., Wang, L. C., Lee, C. K., & Chen, S. H. (2018). Flt3 ligand treatment reduces enterovirus A71 lethality in mice with enhanced B cell responses. Scientific Reports, 8(1). https://doi.org/10.1038/s41598-018-30631-2
Pham, T. N. Q., et al. (2019). Flt3L-Mediated Expansion of Plasmacytoid Dendritic Cells Suppresses HIV Infection in Humanized Mice. Cell Reports, 29(9), 2770-2782.e5. https://doi.org/10.1016/j.celrep.2019.10.094
Remot, A., et al. (2016). Flt3 ligand improves the innate response to respiratory syncytial virus and limits lung disease upon RSV reexposure in neonate mice. European Journal of Immunology, 46(4), 874-884. https://doi.org/10.1002/eji.201545929
Smith, J. R., Thackray, A. M., & Bujdoso, R. (2001). Reduced herpes simplex virus type 1 latency in Flt-3 ligand-treated mice is associated with enhanced numbers of natural killer and dendritic cells. Immunology, 102(3), 352-358. https://doi.org/10.1046/j.1365-2567.2001.01180.x
Solheim, J. C., et al. (2007). Spleen but not tumor infiltration by dendritic and T cells is increased by intravenous adenovirus-Flt3 ligand injection. Cancer Gene Therapy, 14(4), 364-371. https://doi.org/10.1038/sj.cgt.7701018
Tsapogas, P., Mooney, C. J., Brown, G., & Rolink, A. (2017). The cytokine Flt3-ligand in normal and malignant hematopoiesis. In International Journal of Molecular Sciences (Vol. 18, Issue 6). MDPI AG. https://doi.org/10.3390/ijms18061115

## Claims

1. An RNA encoding for an Flt3L protein or a functional fragment thereof for use in enhancing an immune response in a subject.

2. The mRNA or the functional fragment thereof for use according to claim 1, wherein the RNA or the functional fragment thereof is naked RNA, an mRNA molecule, a circular RNA molecule, or a self-amplifying RNA molecule.

3. The mRNA or the functional fragment thereof for use according to claim 1 or 2, wherein the RNA or the functional fragment thereof is part of a lipid nanoparticle (LNP), a polymeric nanoparticle, a polyplex, or a lipoplex, comprising said RNA or the functional fragment thereof.

4. An expression cassette or vector, comprising an RNA and/or a DNA encoding for an RNA, preferably an mRNA, encoding for an Flt3L protein or a functional fragment thereof for use in enhancing an immune response in a subject.

5. The expression cassette or vector for use according to claim 4, wherein the vector is a plasmid vector or a viral vector.

6. A pharmaceutical composition, comprising the RNA or the functional fragment thereof or the LNP for use according to any one of claims 1 to 3, the expression cassette or vector for use according to claim 4 or 5 and at least one pharmaceutically acceptable carrier for use in medicine, in particular for a use in enhancing an immune response in a subject, wherein preferably said composition comprises an aqueous formulation.

7. The pharmaceutical composition for use according to claim 6, wherein said enhancing an immune response in a subject comprises a use as an adjuvant, in particular as an anti-cancer or anti-infective adjuvant, as a suitable adjuvant enhancing immune responses by recruiting DCs and B cells, for an expansion of the numbers of natural killer (NK) cells, DCs and/or T lymphocytes, for priming and expansion of antigen-specific CD8⁺ T cells, an activation of T cells, an increase in cytokine-secreting CD4+ T cells, the expansion of the numbers of natural killer (NK) cells and/or T lymphocytes, for priming and expansion of antigen-specific CD8⁺ T cells and/or for improving the T-cell homing into tumors.

8. The pharmaceutical composition for use according to claim 6, wherein said use comprises a combination prevention or therapy, such as, for example, a pre-treatment to improve vaccines against infectious diseases or cancer, or for enhancing an anti-infective or anticancer treatment in combination with at least one anti-infective or anticancer monoclonal antibody.

9. The pharmaceutical composition for use according to any one of claims 6 to 8, wherein the subject suffers from cancer or infection, in particular bacterial or viral infection.

10. The pharmaceutical composition for use according to any one of claims 6 to 8, wherein the use comprises administration i.v., i.n., and/or i.m., preferably together with another RNA encoding for a protein antigen or adjuvants.

11. A method for enhancing an immune response in a subject, comprising administering an effective amount of an RNA encoding for an Flt3L protein or a functional fragment thereof to said subject, wherein preferably the RNA or the functional fragment thereof is naked mRNA, an mRNA molecule, a circular RNA molecule, or a self-amplifying RNA molecule.

12. The method according to claim 11, wherein the RNA or the functional fragment thereof is part of a lipid nanoparticle (LNP), a polymeric nanoparticle, a polyplex, or a lipoplex, comprising said RNA or the functional fragment thereof.

13. The method according to claim 11 or 12, comprising administering an effective amount of an expression cassette or vector comprising the RNA and/or a DNA encoding for the RNA, preferably the mRNA, encoding for an Flt3L protein or a functional fragment thereof to said subject, wherein preferably the vector is a plasmid vector or a viral vector.

14. The method according to any one of claims 11 to 13, wherein the RNA or the functional fragment thereof, the LNP, the expression cassette or the vector is administered as a pharmaceutical composition, further comprising at least one pharmaceutically acceptable carrier, preferably as an anti-infective or anti-cancer vaccine.

15. The method according to any one of claims 11 to 14, wherein said method comprises the use as an adjuvant, in particular as an anti-cancer or anti-infective adjuvant, as a suitable adjuvant enhancing immune responses by recruiting DCs and B cells, for an expansion of the numbers of natural killer (NK) cells, DCs and/or T lymphocytes, for priming and expansion of antigen-specific CD8⁺ T cells, an activation of T cells, and/or an increase in cytokine-secreting CD4+ T cells, for improving an anti-infective or anti-cancer effect of an intranodal RNA or an RNA-based intranodal vaccine, and/or wherein preferably the use comprises a combination prevention or therapy, such as, for example, a pre-treatment to improve vaccines against infectious diseases or cancer, or for enhancing an anticancer treatment in combination with at least one anticancer monoclonal antibody.
